Europäisches Patentamt

European Patent Office

Office européen des brevets

(19)

(11) **EP 0 574 497 B1**

(12) **FASCICULE DE BREVET EUROPEEN**

(45) Date de publication et mention
de la délivrance du brevet:
**22.05.1996 Bulletin 1996/21**

(21) Numéro de dépôt: **92907249.4**

(22) Date de dépôt: **04.03.1992**

(51) Int Cl.⁶: **G01N 33/18**, G01N 33/00

(86) Numéro de dépôt international:
**PCT/FR92/00194**

(87) Numéro de publication internationale:
**WO 92/15877 (17.09.1992 Gazette 1992/24)**

(54) **PROCEDE ET DISPOSITIF DE DOSAGE D'UN COMPOSE PRESENT DANS UN MILIEU LIQUIDE, AYANT UNE FORME VOLATILE BASIQUE**

**VERFAHREN UND VORRICHTUNG ZUR MESSUNG EINER FLÜCHTIGEN, BASISCHEN VERBINDUNG IN EINEM FLÜSSIGEN MEDIUM**

**METHOD AND DEVICE FOR MEASURING A COMPOUND CONTAINED IN A LIQUID MEDIUM, HAVING AN ALCALINE VOLATILE FORM**

(84) Etats contractants désignés:
**BE CH DE ES GB IT LI NL**

(30) Priorité: **08.03.1991 FR 9103145**

(43) Date de publication de la demande:
**22.12.1993 Bulletin 1993/51**

(73) Titulaire: **AIR ET EAU INGENIERIE (S.A.)**
**F-34830 Clapiers (FR)**

(72) Inventeurs:
• **GAL, Jean-Yves**
**F-34090 Montpellier (FR)**

• **BOURG, Bernard Rés. Le Blason**
**F-34090 Montpellier (FR)**

(74) Mandataire: **Moretti, René et al**
**c/o Cabinet Beau de Lomenie,**
**232, Avenue du Prado**
**F-13295 Marseille (FR)**

(56) Documents cités:
**EP-A- 0 359 158          DE-A- 3 842 068**

• **PATENT ABSTRACTS OF JAPAN vol. 10, no. 282 (P-500)(2338) 25 Septembre 1986**

## Description

La présente invention a pour objet un procédé et dispositif de dosage d'un composé présent dans un milieu liquide et ayant une forme volatile basique.

Le secteur technique de l'invention est le domaine de la fabrication et de l'exploitation d'analyseurs de produits en solution comportant en particulier un capteur de mesure permettant le dosage d'un produit secondaire de réaction, et donc indirectement celui du produit spécifique que l'on veut doser.

Une des applications principales de l'invention est de pouvoir doser l'azote ammoniacal contenu dans les eaux. En effet l'azote ammoniacal traduit un processus de dégradation incomplète de la matière organique et participe de ce fait à l'eutrophisation des milieux aquatiques; ainsi en ce qui concerne les eaux usées et le milieu naturel, il représente un excellent indicateur de niveau de pollution; des normes sont du reste en vigueur, et en particulier en France où la concentration maximale admissible (C.M.A.) est fixée à 0,5 mg/litre à ce jour.

Cette application principale permet ci-après de décrire et d'expliquer le procédé et le dispositif suivant l'invention mais il est clair et évident, pour tout homme du métier dans le domaine du dosage, que ces procédés et dispositifs pourraient être utilisés pour le dosage de tout autre produit ayant une forme chimique volatile basique, tel que les amines volatiles et présent dans un milieu liquide, et donc applicables dans d'autres domaines que celui du contrôle de la pollution des eaux.

Cependant pour présenter et mieux décrire l'invention on s'appuiera essentiellement ci-après d'une part sur le produit à doser à titre d'exemple, qui est l'azote ammoniacal, rentrant dans la catégorie générale des produits chimiques ayant une forme volatile basique et sur lequel de nombreux développements existent, et d'autre part sur son application principale dans le contrôle des eaux.

On connaît en effet différentes techniques pouvant être utilisées pour le dosage de produits volatils basiques, tels que l'azote ammoniacal, et que l'on peut classer en trois grandes catégories principales:

- La première catégorie regroupe les méthodes colorimétriques qui font appel à des réactifs que l'on introduit dans la solution contenant le produit à doser et qui étant en quantités connues colorent plus ou moins la solution suivant la réaction avec ledit produit à doser: la mesure de cette coloration en comparaison avec des colorations de solutions étalons permet ensuite de connaître la quantité de réactif, qui s'est combiné avec ce produit, et donc indirectement de calculer le dosage de ce dernier dans la solution initiale. Ces méthodes sont cependant très perturbées par une turbidité éventuelle de la solution, ce qui est souvent le cas en milieu naturel, par une coloration propre de cette solution et par la présence d'autres produits pouvant réagir avec le réactif: ce dernier point peut-être corrigé par l'emploi de plusieurs réactifs qui permettent de sélectionner le produit recherché, et par des mises en températures données pour améliorer cette sélection, mais cela rend difficile l'opération et ne donne pas de résultats très fiables, beaucoup d'interférences persistant.

Dans le domaine de l'azote ammoniacal, les réactions colorimétriques peuvent être suivant les méthodes classiques connus de BERTHELOT et de NESSLER, telles que décrites en particulier dans les publications de la Norme Française, éditée par l'AFNOR, référencée NF-T-90015 d'Août 1975 et homologuée par arrêté du 30 Juillet 1975 publiée dans le J.O. du 13 Août 1975 en deuxième et troisième parties: la deuxième partie est intitulée "méthode spectrophotométrique au bleu d'indophénol" (méthode de BERTHELOT) et la troisième "méthode spectrophotométrique au réactif de NESSLER".

Dans un domaine plus général de mesure de dosage par réactifs colorimétriques, on relève également le brevet américain US 3998591 déposé le 26 Septembre 1975 pour E.L. ECKFELDT, pour la société LEEDS & NORTHROP COMPANY et décrivant un analyseur spectrochimique comportant un capteur transparent que l'on immerge dans le liquide comportant le produit à doser, et dont la surface est recouverte d'un réactif colorimétrique.

- La deuxième catégorie concerne les méthodes d'acidimétrie sélective à diffusion gazeuse qui utilisent le caractère volatil basique du produit à doser en provoquant l'évaporation de ce produit sous une forme gazeuse: en particulier on rajoute un produit fortement basique pour faire augmenter la teneur en ions OH- de la solution jusqu'à ce que, par exemple pour l'azote ammoniacal le $pH \geq 12$ en utilisant à titre d'exemple de la soude, on obtienne des conditions telles que tout le produit soit gazeux, soit pour l'azote ammoniacal $NH_4^+ + OH^- \longrightarrow NH_3 + H_2O$, à 100% de réaction.

Ce gaz est alors récupéré pour être redissous dans une solution dont le titre acide est connu: la variation de pH (car dans le cas de l'ammoniac $NH_3 + H^+ \longrightarrow NH_4^+$) est donc mesurée et permet comme pour la mesure de couleur dans les premières méthodes de connaître indirectement le dosage du produit initial.

Une méthode d'acidimétrie après distillation est décrite pour l'azote ammoniacal dans la publication de la Norme française éditée par l'AFNOR suivant les références précédentes et en première partie. Cette méthode est très perturbée par tout autre produit acide ou basique volatil existant également dans le milieu aqueux d'origine, dans lequel on veut faire le dosage du seul produit auquel on s'intéresse; ainsi dans le cas de l'azote ammoniacal, l'analyse est perturbée par exemple par les amines, et par les produits hydrolysables en ammoniac, tels que l'urée.

Pour tenter d'éviter ou de limiter ces interférences, de nombreuses méthodes utilisent des membranes sélectives qui ne laissent passer que certaines molécules: on les interpose entre la solution initiale du milieu aqueux d'origine dans laquelle on provoque l'évaporation du produit à doser sous sa forme gazeuse, et la solution de titre acide connu, dans laquelle est redissoute ladite forme gazeuse après avoir diffusé à travers la membrane. La mesure de pH dans cette solution réceptrice se fait alors en général par une mesure de tension entre deux électrodes immergées dans cette solution.

On peut citer parmi ces méthodes d'acidimétrie à électrodes sélectives et à diffusion gazeuse, le brevet allemand DD 201206 déposé le 18 Novembre 1981 par GERSTMEYER A. et autres pour VEB PETROLCHEMISCHES KOMBINAT SCHNEDT sur un procédé de détermination automatique et en continu de la concentration d'ammoniac dans un fluide.

Les problèmes de ces méthodes à membrane sont multiples: d'une part il y a colmatage des circuits et surtout de la membrane à cause des autres molécules qui ne peuvent pas la traverser et qui ont été générées par les réactions d'autres produits de la solution d'origine avec le produit fortement basique introduit; ainsi la membrane doit être changée fréquemment, au moins tous les 2 ou 3 jours; d'autre part l'étalonnage des solutions réceptrices est difficile, et la reproductibilité n'est donc pas assurée; enfin la mesure potentiométrique du pH est une mesure, par conception, non proportionnelle à la concentration, puisqu'en ce cas la réponse n'est proportionnelle qu'au logarithme de celle-ci, ce qui accentue l'imprécision du dosage obtenu et donne des résultats que l'on peut considérer comme assez mauvais.

- Enfin la troisième catégorie regroupe les méthodes à chambre de diffusion gazeuse et à analyse spectrophotométrique qui sont en fait la combinaison des deux types de méthodes précédentes pour essayer de pallier aux problèmes non résolus par celles-ci et à leurs inconvénients.

Pour cela on fait également diffuser le gaz produit dans la solution à analyser à travers une membrane, mais de l'autre côté on rajoute un indicateur coloré dans la solution réceptrice afin d'obtenir un changement de couleur par réaction avec le gaz redissous: on peut alors mesurer par analyse spectrophotométrique la couleur obtenue, ce qui permet de connaître la quantité de gaz ayant traversé la membrane et donc de calculer le dosage initial du produit; cette mesure colorimétrique est plus sensible que la mesure de pH, : elle permet également une meilleure répétitivité et reproductibilité ainsi qu'un meilleur étalonnage.

Des exemples d'utilisation de ces méthodes sont décrits et connus dans divers documents tels que dans les deux publications de 1983 - 153 (Pages 271-275) et 1988 - 208 (Pages 81-90) de la revue Analytica Chimica Acta, dont la première est de Monsieur VAN SON et al, et la deuxième de Monsieur RYUJI NAKATA et al: ces publications décrivent des applications particulières de ces méthodes avec des essais d'optimisation de certains paramètres (débits, températures, structures des membranes...) et des possibilités de correction pour en améliorer les résultats.

En effet ces méthodes, bien que meilleures que les précédentes comportent toujours des inconvénients dont les deux plus importants sont:

- d'une part le colmatage rapide de la membrane, qui de plus ne laissant passer de toute façon que 20 % environ du gaz formé permet d'autant moins une bonne répétabilité et stabilité des mesures quand ce pourcentage varie du fait de ce colmatage; ce qui est un inconvénient déjà évoqué dans les méthodes précédentes à membrane.

- et d'autre part, ces méthodes ne permettent pas facilement d'éliminer le gaz carbonique ($CO_2$) toujours présent dans l'air et particulièrement gênant au niveau de la mesure spectrophotométrique - surtout si une sensibilité élevée est souhaitée - car il traverse également la membrane et réagit avec l'indicateur coloré de pH comme la forme gazeuse que l'on veut doser, masquant ainsi en partie le titrage de cet indicateur. Il est alors nécessaire de dégazer ce gaz carbonique et de rester ensuite en milieu d'azote neutre, mais cela complique les opérations et ne permet pas de garantir de toute façon l'exactitude des mesures. Ceci est vrai même en utilisant des méthodes de correction par calcul, telles que décrites dans les documents cités de la revue ACA ci-dessus, où le calcul est utilisé pour faire les mesures sans dégazer, ce qui est délicat puisque la solubilité de $CO_2$ peut varier (influence de la température notamment), puis les auteurs comparent leurs résultats à une méthode de référence (BERTHELOT).

Le problème posé est donc de trois ordres afin d'obtenir de nouvelles méthodes et dispositifs de dosage en continu d'un composé présentant la propriété d'avoir une forme volatile basique et présent dans un milieu liquide, qui sont que :

- il n'y ait pas d'interférences, ou tout au moins le moins possible du fait de la présence d'autres composés ayant une forme volatile ayant des caractéristiques gênantes pour la mesure, tels qu'essentiellement l'acide carbonique, dans ce milieu liquide et/ou dans le système,
- il n'y ait pas de colmatage du circuit nécessitant des interventions fréquentes et diminuant la fiabilité de la mesure,
- on puisse effectuer cette mesure de dosage in situ d'une façon qui puisse être automatisée et avec une sensibilité et une précision suffisantes conformes aux normes imposées.

Une solution au problème posé est un procédé de dosage d'un composé présentant la propriété d'avoir une forme volatile basique et présent dans un milieu liquide que l'on introduit dans un réservoir, chambre ou cellule avec un produit basique de réaction afin de volatiliser dans ladite cellule la forme gazeuse basique dudit produit, laquelle forme gazeuse est ensuite redissoute dans une solution réceptrice contenant un indicateur donné afin de pouvoir mesurer par tout système connu la valeur d'un paramètre déterminé qui peut être fonction de la valeur de pH de ladite solution et qui varie suivant la redissolution de ladite forme gazeuse, et permettant d'en déduire ledit dosage, tel que:

- on place ladite solution réceptrice dans au moins un deuxième réservoir, chambre ou cellule, étanche et dont on connaît et mesure à l'état initial la valeur dudit paramètre, et on injecte par tout moyen reliant la cellule à la cellule suivante le gaz volatilisé dans la partie supérieure de la première cellule dans ladite solution réceptrice contenue dans la partie inférieure de la cellule suivante, plusieurs cellules de redissolution pouvant être placées en série,
- on fait recirculer en circuit fermé le gaz récupéré dans le volume supérieur de la dernière cellule, vers la première cellule, et on l'injecte dans le milieu liquide qu'elle contient dans son volume inférieur,
- on effectue la mesure de la valeur dudit paramètre modifiée, par rapport à l'état initial, par la dissolution de ladite forme gazeuse dans la solution réceptrice de la dernière cellule, après avoir effectué un nombre de boucles de recirculation permettant d'obtenir un rapport de concentration, suffisant pour pouvoir exploiter la mesure, entre la concentration initiale du produit à doser dans la solution initiale et celle de dissolution du produit considéré dans la solution réceptrice.

Une autre solution au problème posé est un dispositif de dosage d'un composé présentant la propriété d'avoir une forme volatile basique et présent dans un milieu liquide, comprenant un réservoir ou cellule dans lequel est introduit ledit liquide avec un produit basique de réaction afin de volatiliser dans ladite cellule la forme gazeuse dudit produit, laquelle forme gazeuse est ensuite redissoute dans une solution réceptrice contenant un indicateur permettant d'en déduire à partir d'un paramètre connu la valeur du dosage recherché grâce à tout système de mesure correspondant et compris dans ledit dispositif, tel que celui-ci comprend en outre au moins un deuxième réservoir ou cellule dans lequel est introduit ladite solution réceptrice dont est connue la valeur dudit paramètre caractéristique du dosage recherché et mesurable par ledit système de mesure associé à cette au moins deuxième cellule et un circuit fermé étanche de recirculation du gaz récupéré dans le volume supérieur de la au moins deuxième cellule vers et dans le milieu liquide contenu dans le volume inférieur de la première cellule, et un moyen d'injection du gaz volatilisé et celle-ci vers et dans le milieu liquide de la cellule suivante.

Compte tenu des caractéristiques principales de l'invention, telles que définies ci-dessus, l'indicateur donné permettant de quantifier le dosage recherché est préférentiellement un indicateur de pH, et le paramètre mesurable pour en déduire ce dosage est alors le résultat du titrage de cet indicateur de pH par ladite forme gazeuse basique.

Dans un mode de réalisation préférentiel on introduit dans le milieu liquide, et ceci d'autant plus si ce milieu contient également un composé risquant d'interférer, un produit basique en quantité suffisante pour obtenir une valeur de pH de ce milieu au moins égale à la valeur du pKA + 1 dudit composé que l'on veut doser, et on choisit une solution réceptrice ayant une valeur de pH inférieure au pKA-1 le plus faible du composé interférant potentiel tel que l'acide carbonique tout en restant compatible avec la zone de virage de l'indicateur coloré de pH éventuellement utilisé.

De préférence également on utilise un indicateur qui se colore en fonction de la valeur de pH de la solution réceptrice et on mesure la couleur obtenue avec un détecteur spectrophotométrique, lequel détecteur spectrophotométrique peut être monté directement autour de la cellule de redissolution et est constitué d'une barrette de diodes permettant une détection et une mesure multi-longueurs d'onde de la variation de couleur dudit indicateur.

Dans un mode de réalisation préférentiel l'ensemble du dispositif suivant l'invention est étanche et chaque cellule est d'un volume parfaitement défini et les volumes inférieurs desdites cellules contiennent des quantités de milieu liquide constantes connues, contrôlées par des évacuations à niveau constant et des alimentations à débit contrôlable.

Le résultat est de nouveaux procédés et dispositifs de dosage en continu de composés présentant la propriété d'avoir une forme volatile basique et présents dans un milieu liquide, et plus spécialement d'azote ammoniacal dans les eaux qui peuvent être potables, piscicoles, usées ou d'un milieu naturel.

Les procédés et dispositifs répondent aux inconvénients cités dans les méthodes décrites précédemment et aux problèmes posés, alors qu'à ce jour de nombreux autres développements de techniques de dosage sont étudiés sans obtenir les mêmes caractéristiques de qualité et de fiabilité: on peut citer par exemple les méthodes indirectes utilisant des composés fluorescents, l'électrode biologique à membrane bactérienne, l'électrode à membrane sélective des ions ammonium NHa+ à bicouche lipidique, les méthodes physico-chimiques utilisant des semiconducteurs...

Les méthodes enzymatiques donnent de bons résultats mais présentent l'inconvénient de nécessiter un appareillage complexe et coûteux, ce qui en restreint l'utilisation aux applications médicales.

Les procédés et dispositifs suivant l'invention permettent en effet de pouvoir piéger tout produit volatil acide gênant dans la première cellule du dispositif, et de faire une mesure fiable dans la deuxième cellule car il n'y a plus alors de risque d'interférence lorsqu'une détection à mesure directe ou indirecte de pH est utilisée. Ceci peut être le cas si la solution initiale contenant le composé à doser contient d'autres composés présentant également une forme volatile : la présente invention peut permettre alors, en disposant plusieurs cellules en série, de piéger ces autres gaz secondaires au fur et à mesure, et éventuellement de pouvoir alors doser également les composés dont ils sont issus suivant un choix de paramètres adaptés. Avec les deux cellules au moins de l'invention, on peut ainsi éliminer au moins un gaz, en particulier le gaz carbonique, et c'est le cas des applications principales de la présente invention, étant donné que les milieux liquides contiennent de manière naturelle de l'acide carbonique qui est très gênant de par ses propriétés acido-basique (2 pKA)

On peut définir simplement le pKA comme étant la valeur du pH de la solution lorsque cette dernière contient autant d'acide que de base d'un même couple acide/base.

En effet,

$$pH = pKA + \log \frac{[Base]}{[Acide]}$$

avec pKA = -log KA,
KA étant la constante d'équilibre de dissociation de l'acide suivant l'expression

$$KA = \frac{[Base]\,[H_3O]^+}{[Acide]}$$

On peut caractériser tout produit volatil basique et/ou acide par un ou plusieurs pKA suivant les formes chimiques qu'il prend en fonction du pH: au delà ou en deçà de certaines valeurs de pH on obtient même une solution ne contenant plus que la (ou les) formes gazeuses, ou que la (ou les) formes ioniques.

Par exemple il est connu que le pKA de l'azote ammoniacal est voisin de 9,25, alors que ceux de l'acide carbonique sont de l'ordre de 6,3 et 10,4 (ces valeurs variant avec la force ionique et la température); la forme gazeuse de l'azote ammoniacal $NH_3$ est en pourcentage plus élevé au dessus de 9,25; et celle de l'acide carbonique en dessous de 6,3, mais entre cette valeur et 10,4, on a un mélange de $CO_2$ acqueux, $HCO_3$- et $CO_3^{--}$, ce qui provoque effectivement des interférences de mesure quand on a en présence ce produit avec un autre ayant une valeur de pKA intermédiaire et dont on veut assurer le dosage, tel que l'azote ammoniacal.

Ainsi dans un exemple de procédé et de dispositif préférentiel suivant l'invention, le $CO_2$ acqueux gênant est rapidement éliminé sous forme de carbonate $CO_3^{--}$ dans la première cellule au cours des premières boucles de recirculation, et la mesure dans la deuxième cellule peut alors être effectuée avec une grande précision, surtout en utilisant les dispositifs préférentiels tels que décrits précédemment: on peut ainsi obtenir une sensibilité de l'ordre de 20 ppb à 50 ppb, alors que la concentration maximale admissible (C.M.A.) pour les eaux potables est actuellement de 500 ppb (0,5 mg/l).

Un autre avantage important de l'invention, nécessaire du reste pour la sensibilité mentionnée ci-dessus, est l'absence de membrane de séparation à travers laquelle les gaz diffusent comme dans les dispositifs actuels: on élimine ainsi tout risque de colmatage, même pour des échantillons d'eaux de duretés courantes, et donc toute diminution de la précision et toute intervention fréquente de nettoyage et de changement de membrane. Ces systèmes utilisant ainsi les procédés et dispositifs suivant l'invention peuvent être installés alors en tout site sans contrôle particulier, avec même un automatisme de prélèvements et de mesure ne nécessitant qu'un minimum d'entretien.

On peut citer du reste d'autres dispositifs et procédés adaptés à divers types de produits soit pour les préparer soit pour les doser, utilisant leurs propriétés également de dissolution et/ou leurs caractères acide et/ou basique et réalisant une circulation de la solution; mais chacun résout des problèmes différents, et aucun ne comporte toutes les caractéristiques essentielles de la présente invention, ne peut répondre au problème posé, et n'est adapté au dosage des composés présentant la propriété d'avoir une forme volatile basique: on note par exemple dans le domaine de la préparation de produit, la demande de brevet FR 2647349 déposée le 29 Mai 1989 par la société HOSPAL Industrie décrivant "un procédé et dispositif pour la préparation d'une solution à usage médical par dissolution de concentrés pulvérulents avec recirculation d'une solution", à partir d'un réservoir où se produit la dissolution comportant d'une part un circuit bouclé sur lui-même et d'autre part un double circuit d'alimentation d'une deuxième zone de mélange où l'on mesure la concentration obtenue que l'on peut alors corriger en régulant le débit de chacun desdits circuits.

On relève également les deux demandes de brevets N° FR 2603699 et FR 2580404 déposées le 3 Novembre 1986 et le 15 Avril 1985 respectivement par l'Université des Sciences et Techniques du Languedoc, toutes deux intitulées "dispositifs pour le dosage ampérométrique en continu des nitrites et/ou des nitrates qui sont présents dans un milieu aqueux quelconque", et décrivant des dispositifs comprenant une cellule à circulation munie d'électrodes de mesure et une cellule de mélange située en amont et dans laquelle on introduit le liquide dont la teneur en nitrite est à déterminer avec une solution tampon.

On peut citer enfin le brevet Fr 2569008 déposé le 16 Mai 1984 par Les Ciments Français sur "un procédé de dosage d'au moins un des constituants d'un composé chimique par dissolution sélective et mesure quantitative du résidu non dissous par détection photométrique".

Tous ces documents décrivent donc des procédés et dispositifs non applicables dans l'objectif de la présente invention même si certains utilisent des moyens communs, connus de toute façon de manière générale mais non suivant la disposition et la combinaison décrites ci-après, tels que les boucles de recirculation, les cellules de mélange, la dissolution de produit, les mesures par électrodes ou par colorimétrie...

La figure 1 est un schéma du procédé et dispositif suivant l'invention.

La figure 2 est un exemple de courbes reliant la concentration de redissolution au nombre de boucles de recirculation.

La figure 3 est un exemple de représentation du dispositif suivant l'invention.

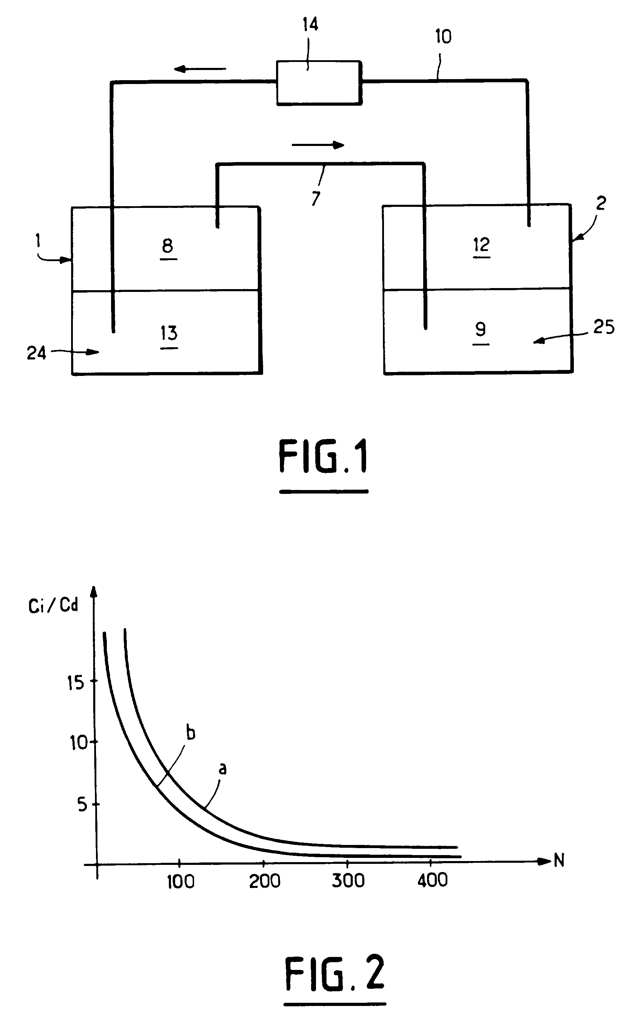

La figure 1 représente un schéma de fonctionnement d'un module permettant de réaliser le procédé suivant l'invention : ce module ou dispositif de volatilisation-redissolution est constitué de deux cellules 1, 2 de faible volume reliées entre elles par un circuit fermé de gaz et d'air 7, 10 qui pour un nombre de boucles de recirculation suffisant assure le transfert du gaz volatilisé dans la cellule 1 vers la seconde 2, tandis que tout produit volatil acide risquant d'interférer tel que le gaz carbonique $CO_2$ est transféré et piégé dans la cellule 1 grâce à un choix déterminé de la valeur de pH des solutions 24 contenue dans la première cellule, et 25 dans la deuxième, combiné également à un choix de température de ces solutions de façon à accélérer et à accentuer le phénomène.

A titre d'exemple de mesures obtenues, on observe qu'on obtient très vite avec une solution à contrôler de concentration initiale $10^{-5}M$ d'azote ammoniacal et contenant de la soude, un rapport de 10 satisfaisant entre cette concentration initiale et la concentration de redissolution après vingt boucles de recirculation environ avec une température de volatilisation dans la cellule 1 de 50°C ou après quarante boucles avec une température de volatilisation dans la cellule 1 de 25°C; la température de redissolution dans la cellule 2 étant fixée dans les deux cas à 25°C. Ces mesures ont été effectuées avec des cellules 1 et 2 contenant chacune 5 ml de solution, respectivement 13 et 9, et 7 ml de volume de gaz, respectivement 8 et 12, et un volume de gaz recirculé 10 de 7 ml.

Dans une application de l'invention, on réalise dans la première cellule 1 la volatilisation forcée de l'azote ammoniacal contenu dans la solution initiale à contrôler 13 par addition de soude dans cette solution. Dans la deuxième cellule 2, on favorise la redissolution du gaz $NH_3$, récupéré depuis la première cellule grâce au circuit d'injection 7 reliant les deux cellules 1 et 2, dans une solution d'indicateur coloré 25 de pH adapté. On détermine le choix de cet indicateur et sa concentration optimale en fonction de la gamme de mesure souhaitée, la variation de couleur de la solution dépendant de la teneur en azote ammoniacal de l'échantillon, dans le cas où on utilise la colorimétrie comme moyen de mesure de ladite concentration.

Dans d'autres modes de réalisation, on pourrait également mesurer le pH de cette solution réceptrice 25 par des électrodes sélectives immergées dans celle-ci et telles que décrites dans divers documents de l'état de la technique connus à ce jour.

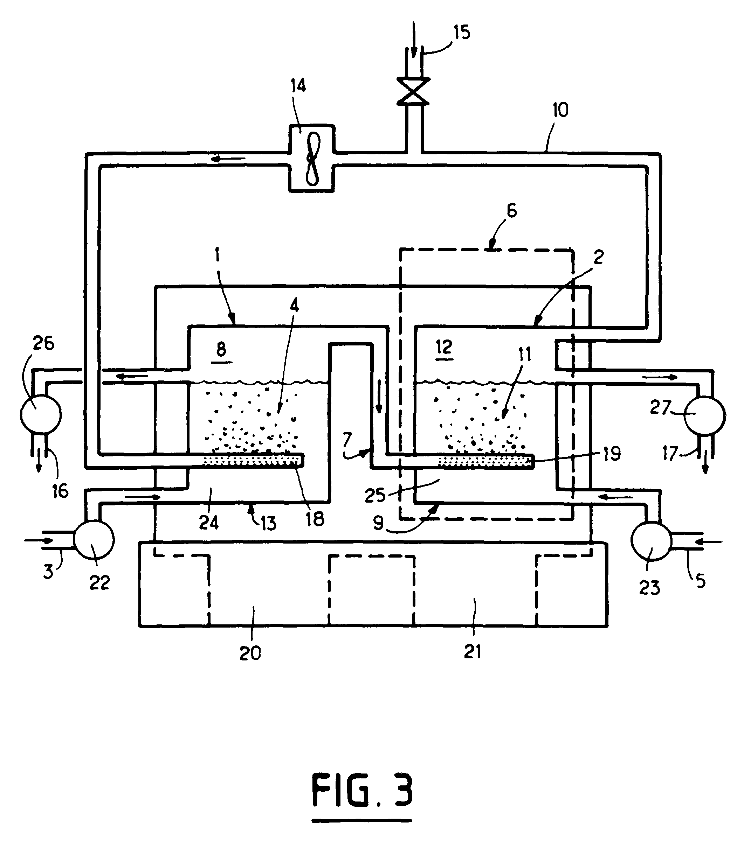

Dans un mode préférentiel, il est retenu plutôt de mesurer ladite concentration grâce à un indicateur qui se colore en fonction de la valeur de pH de la solution réceptrice 25 et on mesure alors la couleur obtenue avec un détecteur spectrophotométrique 6 tel que figuré autour de cette cellule 2 dans la figure 3.

En ce qui concerne cette partie détection et mesure, on note que l'on a une réponse linéaire d'absorbance pour une gamme de concentration donnée de la forme volatile basique de l'azote ammoniacal, en simulant à l'ordinateur les courbes de titrage spectrophotométriques de quelques indicateurs colorés de pH. On peut ainsi retenir plusieurs indicateurs colorés donnant pour l'azote ammoniacal, des résultats tout à fait acceptables, il s'agit du pourpre de bromocrésol, du bleu de bromothymol et du rouge de crésol: le pourpre de bromocrésol à la concentration de $10^{-5}$ M représente le meilleur compromis pour des concentrations de l'ordre de $10^{-6}$ à $10^{-5}$ M en $NH4^+$.

Grâce à un choix judicieux de la valeur de pH de la solution 24 initiale dans laquelle est dissous un produit basique supplémentaire, à savoir une valeur de pH supérieure ou égale au pKA + 1 du composé présentant la propriété d'avoir une forme volatile basique, que l'on veut doser tel que l'azote ammoniacal, on est sûr de volatiliser partiellement ou complètement ladite forme volatile de ce composé. L'intérêt de limiter la valeur de pH à pKA + 1, avec pour conséquence une volatilisation partielle de la forme volatile basique peut s'expliquer par la nécessité d'éviter l'interférence de certaines amines volatiles (méthylamine et diméthylamine) dont les pKA sont respectivement de 10,7 et 10,8. Ainsi pour l'azote ammoniacal dont le pKA est de 9,25 il sera même choisi un pH de la solution 24 égale au moins à 11,25 soit pKA + 2 afin d'être sur de retrouver la totalité de l'azote ammoniacal sous sa forme volatile basique et ainsi d'assurer un rendement de volatilisation optimal.

Par contre, dans la solution réceptrice 25, on choisit une valeur de pH inférieure au pKA le plus faible des interférents potentiels ayant pu suivre le gaz ammoniacal sous leurs formes volatiles acides à travers le circuit d'injection 7, tel qu'en particulier le gaz carbonique que l'on retrouve de manière naturelle dans tous les milieux du fait de sa présence dans l'atmosphère. Sachant que le pKA le plus faible du gaz carbonique est de 6,3, on prend une valeur de pH de cette solution 25 inférieure: par exemple 5,7 qui correspond au pH d'une solution de pourpre de bromocrésol, $10^{-5}$ M. A cette valeur de pH, l'acide carbonique est majoritairement sous forme gazeuse et non plus dissoute, alors que le gaz ammoniacal est lui-même au contraire entièrement dissous et ne peut plus être revolatilisé: ledit gaz carbonique est volatilisé alors dans le volume supérieur 12 de la cellule 2 et peut être entraîné dans la boucle de recirculation 10 grâce à une turbine 14 vers la première cellule 1 où il est injecté dans la solution 24 située dans la partie inférieure 13 de celle-ci pour y être dissous et piégé, du fait de la valeur de pH supérieure de cette solution , telle qu'indiquée ci-dessus, par exemple à 11,25 pour l'azote ammoniacal; dans ce cas, cette valeur correspond en effet à une valeur de pH supérieure au deuxième pKA de l'acide carbonique au-dessus duquel le gaz carbonique est redissous majoritairement sous forme de carbonate.

L'intérêt de ce système est sur ce point particulièrement évident, puisqu'il permet une élimination automatique et systématique de ce gaz carbonique sans rallonger le temps de réponse et sans aucune intervention supplémentaire.

Afin de choisir le produit basique le plus courant dans un mode préférentiel de réalisation du procédé suivant l'invention, il est utilisé de la soude dans la solution 24 afin d'obtenir le pH souhaité dans cette solution. Cette soude peut être éventuellement additionnée avec des composés pouvant régulariser la formation de précipité tel que la calcite : ces composés additifs permettent d'éviter des précipités trop lourds et/ou collants qui adhéreraient aux parois et améliorent la rapidité de fixation du gaz carbonique.

La figure 2 est un exemple de courbes reliant la concentration de redissolution au nombre de boucles de recirculation. En effet, cette courbe indique en abscisses un nombre N de boucles de recirculation à travers le circuit 7 et le circuit 10 de la figure 1. En ordonnées, on figure le rapport entre la concentration initiale Ci du produit à doser 1 dans la solution initiale 24 de la cellule 1, sur la concentration de dissolution Cd dudit produit considéré dans la solution réceptrice 25 de la cellule 2: on s'aperçoit ainsi que le rapport de concentration devient vite acceptable et qu'à partir d'une quarantaine de boucles de recirculation, on a obtenu un rapport de concentration suffisant pour pouvoir exploiter la mesure. On obtient le rapport de concentration optimum de 1 ($Ci/Cd \rightarrow 1$) après 100 à 200 boucles de recirculation, ce nombre variant en fonction de paramètres physiques comme les différents volumes des cellules ou encore la température. Ces mesures ont été faites avec les caractéristiques de cellules telles qu' indiquées précédemment pour le commentaire de la figure 1, et pour deux températures différentes telles que pour la courbe indiquée a, la température de la cellule de volatilisation 1 est de 25°C et pour la courbe b, cette température est de 50°C. En effet, la température de cette cellule de volatilisation peut être avantageusement supérieure à celle de la cellule de redissolution, qui en l'occurrence est maintenue à 25°C dans ces figures, afin d'améliorer la volatilisation en ce cas de l'ammoniac dans la cellule 1 et diminuer le nombre de boucles de recirculation nécessaires pour atteindre un rapport de concentration compatible avec le seuil de détection souhaité. En parallèle, comme indiqué précédemment, le $CO_2$ est éliminé sous forme de carbonate dans la cellule de volatilisation au cours des premières boucles de recirculation, les deux phénomènes ayant lieu simultanément et en complément, la mesure dans la deuxième cellule de la concentration Cd peut être faite à partir d'un nombre de boucles déterminé suivant le niveau de précision souhaité.

La figure 3 est un exemple de représentation du dispositif suivant l'invention tel que schématisé dans la figure 1 et suivant les procédés tels que repris ci-dessus. On retrouve dans cette figure le dispositif de dosage en continu d'un composé présentant la propriété d'avoir une forme volatile basique et présent dans un milieu liquide 24 avec un produit basique de réaction afin de volatiliser dans ladite cellule 1 la forme gazeuse 4 dudit produit; cette forme gazeuse est ensuite redissoute dans une solution réceptrice 25 contenant un indicateur permettant d'en déduire à partir d'un paramètre connu la valeur du dosage recherché grâce à tout système de mesure 6 correspondant et compris dans ledit dispositif. Celui-ci comprend en outre au moins un deuxième réservoir ou cellule 2 dans lequel est introduit ladite solution réceptrice 25 dont est connue la valeur dudit paramètre caractéristique du dosage recherché et mesurable par ledit système de mesure 6, associé à cette au moins deuxième cellule 2 et un circuit fermé étanche 10 de recirculation du gaz récupéré 11 dans le volume supérieur 12 de la au moins deuxième cellule 2 vers et dans le milieu liquide 24 contenu dans le volume inférieur 13 de la première cellule 1, et un moyen d'injection 7 du gaz 4 volatilisé dans celle-ci vers et dans le milieu liquide 25 de la cellule suivante.

Ledit moyen 7 d'injection relie le volume supérieur 8 de la cellule 1 au volume inférieur 9 de la suivante et comporte un tube poreux 19 débouchant dans la solution réceptrice 25, à travers laquelle bulle le gaz qui y est amené.

Par ailleurs, le circuit fermé étanche de recirculation 10 comprenant la turbine 14 permettant d'assurer ladite recirculation peut comprendre également une entrée d'air, ou de tout gaz inerte de préférence, extérieure 15 grâce à une vanne étanche de fermeture de façon à pouvoir renouveler le mélange gazeux situé dans le système entre deux séries de mesures.

L'ensemble du dispositif doit être étanche et chaque cellule 1 et 2 est un volume parfaitement défini et les volumes inférieurs 13 et 9 desdites cellules contiennent des quantités de milieux liquides 24 et 25 constants connus, contrôlées par des évacuations 16 et 17 à niveau constant et des alimentations 3 et 5 à débit contrôlable.

Ces divers volumes, comme indiqué déjà ci-dessus à titre d'exemple, peuvent être très réduits donc de l'ordre de quelques ml (2 à 10 ml) en ce qui concerne les volumes supérieurs et inférieurs desdites cellules. Pour assurer cesdits volumes constants connus et l'étanchéité de l'ensemble des cellules et du dispositif, lesdites évacuations 16, 17 et lesdites alimentations 3 et 5 comportent des pompes péristaltiques assurant d'une part l'étanchéité des circuits et d'autre part le contrôle des volumes des milieux liquides 24 et 25: cesdites pompes péristaltiques sont repérées sur la figure 3 suivant les références 22, 23 ,26 et 27, et permettant une mesure en continu en faisant circuler l'échantillon à doser en permanence. Pour avoir cependant une mesure précise de la valeur du paramètre à contrôler, on peut arrêter la circulation de cet échantillon puis après plusieurs boucles de recirculation, celle du gaz récupéré 11 dans le circuit fermé 10. On peut également continuer la circulation de l'échantillon et arrêter seulement la recirculation du gaz pour la mesure.

Le circuit de recirculation 10 qui boucle la deuxième cellule avec la première peut se terminer également par un tube poreux 18 du même type que celui décrit pour la cellule 2 afin de faire buller le gaz récupéré dans la deuxième cellule dans la première avec un maximum de redissolution lors des premières boucles de passage.

Pour assurer également une meilleure homogénéité de cette dissolution du gaz par bullage dans lesdites cellules, chacune ou l'ensemble peut être monté sur des agitateurs de type magnétique 20, 21.

Comme indiquée précédemment, la mesure est réalisée grâce à un détecteur 6 monté sur la cellule 2 placé de telle sorte que la mesure est faite directement dans cette cellule et de préférence par un système de mesure qui est un détecteur spectrophotométrique quand l'indicateur entraîne une coloration (BERTHELOT ou NESSLER) ou change de coloration en fonction de la variation du pH de la solution réceptrice 25 qui varie suivant la quantité de la forme gazeuse 4 qui y est dissous à travers le moyen 7 d'injection.

Pour favoriser la mesure d'absorbance effectuée par cedit système de mesure, on peut rajouter dans la solution réceptrice 25 de l'acide conjugué du composé présentant la propriété d'avoir une forme volatile basique que l'on veut doser tel que pour l'azote ammoniacal, des ions ammonium $NH_4+$ sous forme de chlorure d'ammonium $NH_4CL$ permettant, si nécessaire, d'élargir la gamme de mesure mais en perdant en précision.

De plus le suivi du titrage de cet indicateur coloré de pH peut être réalisé par un suivi indirect de ce pH à l'aide d'un logiciel faisant intervenir les absorbances aux longueurs d'onde maximum de la forme acide et de la forme basique de cet indicateur coloré et une absorbance pour une longueur d'onde dite de confirmation où ces deux formes n'absorbent pas: ceci permet de corriger les éventuelles variations d'absorbance dues à l'appareillage qu'il convient donc d'éliminer. Pour obtenir une telle analyse, il est souhaitable d'utiliser un détecteur à barrette de diodes permettant une mesure multi-longueurs d'onde desdites formes acide et basique pour déterminer avec une plus grande précision la quantité de gaz dissous, en particulier tel que l'ammoniac qui titre la forme acide de l'indicateur coloré utilisé. Cette mesure indirecte de pH est directement proportionnelle à la concentration du gaz dissous et permet ainsi une mesure très précise.

Dans d'autres modes de réalisation éventuels, il peut être combiné plusieurs cellules telles que plusieurs cellules 2 de redissolution les unes derrière les autres avec plusieurs systèmes d'injection 7 reliant la précédente à la suivante et une seule boucle de recirculation 10 reliant la dernière à la première. Ceci permettrait de répartir dans chacune de ces cellules 2 successives différents types de réactifs et de volatiliser par réactions successives différents types de gaz de façon à réaliser plusieurs dosages différents avec un même dispositif. Ceci peut permettre également, sans chercher à en mesurer les dosages, d'éliminer toutes formes volatiles de composés pouvant interférer et qui existeraient dans la solution initiale, en plus de l'élimination du gaz carbonique et tel que cela a été décrit précédemment.

## Revendications

1. Procédé de dosage d'un composé présentant la propriété d'avoir une forme volatile basique et présent dans un milieu liquide (24) que l'on introduit (3) dans un réservoir, chambre ou cellule (1) avec un produit basique de réaction afin de volatiliser dans ladite cellule (1) la forme gazeuse (4) dudit produit, laquelle forme gazeuse est ensuite redissoute dans une solution réceptrice (25) contenant un indicateur donné afin de pouvoir mesurer par tout système connu (6) la valeur d'un paramètre déterminé qui peut être fonction de la valeur de pH de ladite solution (25) et qui varie suivant la redissolution de ladite forme gazeuse, et permettant d'en déduire ledit dosage caractérisé en ce que :

   - on place ladite solution réceptrice (25) dans au moins un deuxième réservoir, chambre ou cellule (2), étanche et dont on connaît et mesure à l'état initial la valeur dudit paramètre, et on injecte par tout moyen (7) reliant la cellule (1) à la cellule suivante (2) le gaz volatilisé dans la partie supérieure (8) de la première cellule (1) dans ladite solution réceptrice (25) contenue dans la partie inférieure (9) de la cellule suivante (2),
   - on fait recirculer en circuit fermé (10) le gaz récupéré (11) dans le volume supérieur (12) de la dernière cellule (2), vers la première cellule (1), et on l'injecte dans le milieu liquide (24) qu'elle contient dans son volume inférieur (13),
   - on effectue la mesure de la valeur dudit paramètre modifiée, par rapport à l'état initial, par la dissolution de ladite forme gazeuse dans la solution réceptrice (25) de la dernière cellule (2) après avoir effectué un nombre de boucles de recirculation permettant d'obtenir un rapport de concentration, suffisant pour pouvoir exploiter la mesure, entre la concentration initiale du produit à doser dans la solution initiale (24) et celle de dissolution du produit considéré dans la solution réceptrice (25).

2. Procédé suivant la revendication 1 caractérisé en ce qu'on introduit dans le milieu liquide (24), qui contient également un composé risquant d'interférer, ledit produit basique en quantité suffisante pour obtenir une valeur de pH de ce milieu supérieure ou égale à la valeur du pKA + 1 dudit composé que l'on veut doser, et on choisit une solution réceptrice (25) ayant si possible une valeur de pH inférieure au pKA-1 le plus faible du composé interférent potentiel.

3. Procédé suivant l'une quelconque des revendications 1 à 2 caractérisé en ce qu'on utilise comme produit basique de la soude.

4. Procédé suivant l'une quelconque des revendications 1 à 3 caractérisé en ce qu'on effectue ladite mesure de la valeur du paramètre en arrêtant la recirculation du gaz récupéré (11) dans le circuit fermé (10).

5. Procédé de dosage suivant l'une quelconque des revendications 1 à 4 caractérisé en que l'on utilise un indicateur qui se colore en fonction de la variation de pH de la solution réceptrice (25) et on mesure la couleur obtenue avec un détecteur spectrophotométrique (6).

6. Procédé suivant l'une quelconque des revendications 1 à 5 caractérisé en ce qu'on peut rajouter dans la solution réceptrice (25) de l'acide conjugué du composé que l'on veut doser, afin d'élargir la gamme de mesure.

7. Dispositif de dosage d'un composé présentant la propriété d'avoir une forme volatile basique et présent dans un milieu liquide (24), comprenant un réservoir ou cellule (1) dans lequel est introduit ledit liquide (24) avec un produit basique de réaction afin de volatiliser dans ladite cellule (1) la forme gazeuse (4) dudit produit, laquelle forme gazeuse est ensuite redissoute dans une solution réceptrice (25) contenant un indicateur permettant d'en déduire à partir d'un paramètre connu la valeur du dosage recherché grâce à tout système de mesure (6) correspondant et compris dans ledit dispositif, caractérisé en ce que celui-ci comprend en outre au moins un deuxième réservoir ou cellule (2) dans lequel est introduit ladite solution réceptrice (25) dont est connue la valeur dudit paramètre caractéristique du dosage recherché et mesurable par ledit système de mesure (6), associé à cette au moins deuxième cellule (2) et un circuit fermé étanche (10) de recirculation du gaz récupéré (11) dans le volume supérieur (12) de la au moins deuxième cellule (2) vers et dans le milieu liquide (24) contenu dans le volume inférieur (13) de la première cellule (1), et un moyen d'injection (7) du gaz (4) volatilisé dans celle-ci vers et dans le milieu liquide (25) de la cellule suivante.

8. Dispositif de dosage suivant la revendication 7 caractérisé en ce que ledit indicateur se colore en fonction de la valeur de pH de la solution réceptrice (25) qui varie suivant la quantité de la forme gazeuse (4) qui y est dissous à travers le moyen (7), et ledit système de mesure (6) est alors un détecteur spectrophotométrique.

9. Dispositif de dosage suivant la revendication 8 caractérisé en ce que ledit détecteur spectrophotométrique (6) est monté directement autour de la cellule (2) de redissolution et est constitué d'une barrette de diodes permettant une détection et une mesure multi-longueurs d'onde de la coloration dudit indicateur.

10. Dispositif de dosage suivant l'une quelconque des revendications 8 et 9 caractérisé en ce que l'ensemble du dispositif est étanche et chaque cellule (1, 2) est d'un volume parfaitement défini et les volumes inférieurs (13, 9) desdites cellules contiennent des quantités de milieu liquide (24, 25) constants connus, contrôlées par des évacuations (16, 17) à niveau constant et des alimentations (3, 5) à débit contrôlable.

11. Dispositif de dosage suivant la revendication 10 caractérisé en ce que lesdites évacuations (16, 17) et lesdites alimentations (3, 5) comportent des pompes péristaltiques assurant d'une part l'étanchéité des circuits et d'autre part le contrôle des volumes des milieux liquides (24, 25).

12. Dispositif de dosage suivant l'une quelconque des revendications 8 à 11 caractérisé en ce que ledit moyen (7) d'injection relie le volume supérieur (8) de la cellule (1) au volume inférieur (9) de la suivante et comporte un tube poreux (19) débouchant dans la solution réceptrice (25), à travers laquelle bulle le gaz qui y est amené.

**Patentansprüche**

1. Verfahren zur quantitativen Bestimnung einer Verbindung, die in einer basischen flüchtigen Form vorliegen kann und in einem flüssigen Medium (24) enthalten ist, das in einen Behälter, eine Kammer oder eine Zelle (1) mit einer zur Umsetzung dienenden basischen Verbindung eingebracht wird (3), um in der Zelle (1) die flüchtige gasförmige Verbindung (4) freizusetzen und diese anschließend in einer Aufnahmelösung (25) wieder zu lösen, die einen bestimmten Indikator enthält, um mit einem beliebigen bekannten System (6) den Wert eines bestimmten Parameters messen zu können, der vom pH-Wert der Lösung (25) abhängen kann und sich je nach der Wiederauflösung cier gasförmigen Verbindung ändert und auf d.essen Basis die quantitative Bestimmung durchgeführt werden kann, **gekennzeichnet durch** folgende Schritte:

- Einbringen der Aufnahmelösung (25) in mindestens einen zweiten Behälter, eine zweite Kammer oder eine zweite Zelle (2), die dicht abgeschlossen sind, für die der Wert di.eses Parameters im Anfangszustand bekannt ist und gemessen wird, und Einleiten des im oberen Teil (8) der ersten Zelle (1) verflüchtigten Gases mit Hilfe einer beliebigen Einleiteinrichtung (7), die die Zelle (1) mit der nachgeschalteten Zelle (2) verbindet, in die Aufnahmelösung (25), die in dem unteren Teil (9) der nachgeschalteten Zelle (2) enthalten ist,
- Rückführen im geschlossenen Kreislauf (10) des im oberen Volumen (12) der nachgeschalteten Zelle (2) zurückgewonnenen Gases (11) in die erste Zelle (1) und Einleiten des zurückgewonnenen Gases in das flüssige Medium (24), das die erste Zelle (1) in ihrem unteren Volumen (13) enthält,
- Durchführen der Messung des Wertes des Parameteters, der sich, bezogen auf den Anfangszustand, durch Auflösen Tier gasförmigen Verbindung in der Aufnahmelösung (25) der nachgeschalteten Zelle (2) verändert hat, nachdem das Gas in mehreren Zyklen rückgeführt worden ist, wobei die mehreren Zyklen es ermöglichen, ein für die Auswertung der Messung ausreichendes Konzentrationsverhältnis zwischen der Anfangskonzentration der quantitativ zu bestimmenden Verbindung in der Ausgangslösung (24) und der Konzentration der Lösung der entsprechenden Verbindung in der Aufnahmelösung (25) zu erhalten.

2. Verfahren nach Anspruch 1, dadurch gekennzeichnet, daß in das flüssige Medium (24), das auch eine Verbindung enthält, bei der die Gefahr besteht, daß sie störend wirkt, die basische Verbindung in einer solch großen Menge eingebracht wird, daß ein pH-Wert dieses Mediums erhalten wird, der mindestens so groß ist wie der um 1 erhöhte pKA-Wert der Verbindung, die quantitativ bestimmt werden soll, und daß eine Aufnahmelösung (25) ausgewählt wird, die, wenn möglich, einen pH-Wert aufweist, der kleiner ist als der um 1 verringerte kleinste pKA-Wert der potentiell störenden Verbindung.

3. Verfahren nach einem der Ansprüche 1 und 2, dadurch gekennzeichnet, daß als basische Verbindung Natriumhydroxid verwendet wird.

4. Verfahren nach einem der Ansprüche 1 bis 3, dadurch gekennzeichnet, daß zur Messung des Wertes des Parameters die Rückführung des zurückgewonnenen Gases (11) im geschlossenen Kreislauf (10) gestoppt wird.

5. Verfahren zur quantitativen Bestimmung nach einem der Ansprüche 1 bis 4, dadurch gekennzeichnet, daß ein Indikator verwendet wird, der sich in Abhängigkeit von der Änderung des pH-Werts der Aufnahmelösung (25) färbt, und die erhaltene Farbe mit einem spektralphotometrischen Detektor (6) gemessen wird.

6. Verfahren nach einem der Ansprüche 1 bis 5, dadurch gekennzeichnet, daß der Aufnahmelösung (25) die konjugierte Säure der Verbindung, die quantitativ bestimmt werden soll, zugesetzt wird, um den Meßbereich zu vergrößern.

7. Vorrichtung zur quantitativen Bestimmung einer Verbindung, die in einer flüchtigen basischen Form vorliegen kann und in einem flüssigen Medium (24) enthalten ist, die einen Behälter oder eine Zelle (1) umfaßt, in welche die Flüssigkeit (24) mit einer zur Umsetzung dienenden basischen Verbindung eingebracht wird, um in der Zelle (1) die flüchtige gasförmige Verbindung (4) freizusetzen, die anschließend wieder in eier Aufnahmelösung (25) gelost wird, die einen Indikator enthält, auf dessen Basis es möglich ist, anhand eines bekannten Parameters den Wert der gewünschten quantitativen Bestimmung mit Hilfe eines beliebigen und in der Vorrichtung enthaltenen entsprechenden Meßsystems (6) zu ermitteln, dadurch gekennzeichnet, daß die Vorrichtung außerdem umfaßt:

- mindestens einen zweiten Behälter oder eine zweite Zelle (2), in den/die die Aufnahmelösung (25) eingebracht wird, für die der Wert des charakteristischen Parameters der gewünschten quantitativen Bestimmung bekannt ist und durch das Meßsystem (6) meßbar ist, das mit dieser mindestens zweiten Zelle (2) verbunden ist, und
- einen dicht abgeschlossenen Kreislauf (10) zur Rückführung des in dem oberen Volumen (12) der mindestens zweiten Zelle (2) zurückgewonnenen Gases (11) in das flüssige Medium (24), das in dem unteren Volumen (13) der ersten Zelle (1) enthalten ist, und
- eine Einleiteinrichtung (7) zum Einleiten der in der ersten Zelle (1) freigesetzten gasförmigen Verbindung (4) in das flüssige Medium (25) der nachgeschalteten Zelle.

8. Vorrichtung zur quantitativen Bestimmung nach Anspruch 7, dadurch gekennzeichnet, daß sich der Indikator in Abhängigkeit vom pH-Wert der Aufnahmelösung (25) verfärbt, der mit der Menge der gasförmigen Verbindung (4) variiert, die in dieser Lösung mit Hilfe der Einleiteinrichtung (7) gelöst ist, wobei das Meßsystem (6) dann ein spektralphotometrischer Detektor ist.

9. Vorrichtung zur quantitativen Bestimmung nach Anspruch 8, dadurch gekennzeichnet, daß der spektralphotometrische Detektor (6) unmittelbar um die Zelle (2) herum montiert ist, in der das Gas wieder gelöst wird, und aus einem Diodenarray besteht, mit dem die Erfassung und Messung der Farbe des Indikators bei mehreren Wellenlängen möglich ist.

10. Vorrichtung zur quantitativen Bestimmung nach einem der Ansprüche 8 und 9, dadurch gekennzeichnet, daß die gesamte vorrichtung dicht abgeschlossen ist und jede Zelle (1, 2) ein genau bestimmtes Volumen aufweist und die unteren Volumina (13, 9) dieser Zellen bekannte und konstante Mengen an flüssigem Medium (24, 25) enthalten., die durch Überläufe (16, 17) in konstanter Hohe und Zufuhrleitungen (3, 5) mit kontrollierbarem Durchsatz kontrolliert werden.

11. Vorrichtung zur quantitativen Bestimmung nach Anspruch 10, dadurch gekennzeichnet, daß in den Überläufen (16, 17) und den Zufuhrleitungen (3, 5) Peristaltikpumpen angeordnet sind, die einerseits die Dichtigkeit der Kreisläufe und andererseits die Kontrolle der Volumina der flüssigen Medien (24, 25) sicherstellen.

12. Vorrichtung zur quantitativen Bestimmung nach einem der Ansprüche 8 bis 1.1, dadurch gekennzeichnet, daß die Einleiteinrichtung (7) das obere Volumen (8) der Zelle (1) mit dem unteren Volumen (9) der nachgeschalteten Zelle verbindet und ein poröses Rohr (19) aufweist, das in die Aufnahmelösung (25) mündet, durch die das zugeführte Gas sprudelt.

**Claims**

1. Method for measuring a compound whose property is to have a basic volatile form and which is present in a liquid medium (24) which is introduced (3) into a container, chamber or cell (1) with a basic reaction product in order to volatilize the gaseous form (4) of said product in said cell, which gaseous form is then redissolved in a receiving solution (25) containing a given indicator in order to enable the measurement, by any known system (6) of the value of a predetermined parameter which can be a function of the pH value of said solution (25) and which varies according to the redissolution of said gaseous form, and enabling the deduction thereof of said measurement, characterized in that it consists in:

- placing said receiving solution (25) in at least a second sealed container, chamber or cell (2) of which the value of said parameter is known and measured in the initial state, and in injecting the gas volatilized in the upper part (8) of the first cell (1) by any means connecting the cell (1) to the following cell (2) into said receiving solution (25) contained in the lower part (9) of the following cell (2),
- recirculating, in closed circuit (10), the gas (11) recovered in the upper volume (12) of the last cell (2), towards the first cell (1) and injecting it into the liquid medium (24) which is contained in the lower volume (13) of the first cell,
- measuring the modified value of said parameter which is modified in relation to the initial state, by dissolving said gaseous form in the receiving solution (25) of the last cell (2) after carrying out a number of recirculation loops which make it possible to obtain a concentration ratio sufficient to be able to use the measurement, between the initial solution (24) and that of the dissolution of the product considered in the receiving solution (25).

2. Method according to claim 1, characterized in that said basic product is introduced into the liquid medium (24), which also contains a compound liable to interfere, in sufficient quantity to obtain a pH value of said medium which is higher than or equal to the value of the pKA + 1 of said compound which is to be measured, and a receiving solution (25) is chosen which has, if possible, a pH value lower than the lowest pKA-1 of the potentially interfering compound.

3. Method according to any one of claims 1 to 2, characterized in that caustic soda is used as a basic product.

4. Method according to any one of claims 1 to 3, characterized in that said measurement of the value of the parameter is carried out by stopping the recirculation of the gas recovered (11) in the closed circuit (10).

5. Measuring method according to any one of claims 1 to 4, characterized in that an indicator is used which colours as a function of the variation of pH of the receiving solution (25) and the colour obtained is measured with a spectrophotometric sensor (6).

6. Method according to any one of claims 1 to 5, characterized in that conjugated acid of the compound to be measured can be added to the receiving solution in order to widen the measuring range.

7. Device for measuring a compound with the property of having a basic volatile form and present in a liquid medium (24), comprising a container or cell (1) in which said liquid (24) is introduced with a basic reaction product in order to volatilize in said cell (1) the gaseous form (4) of said product, which gaseous form is then redissolved in a receiving solution (25) containing an indicator which makes it possible to deduce therefrom the sought measurement value on the basis of a known parameter, with the aid of any corresponding measuring system (6) corresponding to and contained in said device, characterized in that said device further comprises at least a second container or cell (2) in which said receiving solution (25) is introduced, said sought characteristic parameter of measurement of which solution is known and can be measured by said measurement system (6) which is associated with said at least second cell (2) and a sealed closed recirculation circuit (10) for the gas (11) recovered in the upper volume (12) of the at least second cell (2) towards and into the liquid medium (24) contained in the lower volume (13) of the first cell (1), and means (7) for injecting the gas volatilized therein towards and into the liquid medium (25) of the following cell.

8. Measuring device according to claim 7, characterized in that said indicator colours as a function of the pH value of the receiving solution (25) which varies according to the quantity of the gaseous form (4) which is dissolved therein via the means (7), and said measurement system (6) is then a spectrophotometric sensor.

9. Measuring device according to claim 8, characterized in that said spectrophotometric sensor (6) is directly mounted around the redissolution cell (2) and comprises a small diode bar enabling the detection and multi-wavelength measurement of the coloration of said indicator.

10. Measuring device according to any one of claims 8 and 9, characterized in that the whole of the device is sealed and each cell (1, 2) has a perfectly defined volume and the lower volumes (13, 9) of said cells contain known constant quantities of liquid medium (24, 25) controlled at a constant level by outlets (16, 17) and by supplies (3, 5) of controllable flow rate.

11. Measuring device according to claim 10, characterized in that said outlets (16, 17) and said supplies (3, 5) comprise peristaltic pumps ensuring on the one hand the scaling of the circuits and on the other hand the control of the volume of the liquid media (24, 25).

12. Measuring device according to any one of claims 8 to 11, characterized in that said injection means (7) connects the upper volume (8) of the cell (1) to the lower volume (9) of the next cell and comprises a porous tube (19) opening into the receiving solution (25), through which the gas which is fed therein bubbles.

## FIG.1

## FIG.2

## FIG. 3